# NEW EUROPEAN PATENT SPECIFICATION

(11) **EP 0 145 166 B2**
(45) Date of publication and mention of the opposition decision: **28.06.1995**
(45) Mention of the grant of the patent: 13.12.1989
(21) Application number: 84306982.4
(22) Date of filing: 12.10.1984
(51) Int. Cl.: A61F 2/00, A61F 5/00, A61L 27/00, B22F 3/00

(54) **Medical device comprising a shape memory alloy**
Medizinische Vorrichtung aus einer Legierung mit Formerinnerungsvermögen (Memory-Legierung)
Dispositif médical comprenant un alliage à mémoire de forme

(30) Priority: 14.10.1983 US 541852
(43) Date of publication of application: 19.06.1985
(73) Proprietor: RAYCHEM CORPORATION (a Delaware corporation), Menlo Park, California 94025 (US)
(72) Inventor: Jervis, James, Atherton California 94025 (US)
(74) Representative: Benson, John Everett

(56) References cited:
- DE-A- 2 802 571
- DE-A- 3 225 151
- US-A- 4 037 324
- US-A- 4 310 354
- Techn. Mitt. Krupp, Forsch.-Ber.34(1976)1,1-16
- Techn. Mitt. Krupp, Forsch.-Ber.37(1979)1,21-34
- Techn. Mitt. Krupp, Forsch.-Ber.40(1982)4,123-134
- Techn. Mitt. Krupp, Forsch.-Ber.35(1977)3,157-171
- Met. Trans 2(1971, 2973-2981)
- Mechanical Engineering (1980)3, 42-43
- Physik in unserer Zeit 8 (1977)2, 33
- Reprint from Science 191(1976) No.4230
- Shape Memory Effects in Alloys, AIME Proceedings, Ed. Perkins, J., Plenum Press, 1975, 29-87, 273-282
- J. of Metals (1980)6, 129-137
- Radiology, 138(1981)1, 37-45
- Materials Engineering (1969)10, 28-31
- NASA-SP 5110, Ch. 7, p.77,79,83,86
- Orthopedic Surgery 32(1981)10,1180 (with English translation)
- Shika-rikouszassihi 23(1982)No.61, 47-57 (with English translation)

## Description

This invention relates to medical devices incorporating shape memory alloys, and to improvements therein.

Materials, both organic and metallic, capable of possessing shape memory are well known. An article made of such materials can be deformed from an original, heat-stable configuration to a second, heat-unstable configuration. The article is said to have shape memory for the reason that, upon the application of heat alone, it can be caused to revert, or to attempt to revert, from its heat-unstable configuration to its original, heat-stable configuration, i.e. it "remembers" its original shape.

Among metallic alloys, the ability to possess shape memory is a result of the fact that the alloy undergoes a reversible transformation from an austenitic state to a martensitic state with a change in temperature. This transformation is sometimes referred to as a thermoelastic martensitic transformation. An article made from such an alloy, for example a hollow sleeve, is easily deformed from its original configuration to a new configuration when cooled below the temperature at which the alloy is transformed from the austenitic state to the martensitic state.

The temperature at which this transformation begins is usually referred to as Mₛ and the temperature at which it finishes M_{f} When an article thus deformed is warmed to the temperature at which the alloy starts to revert back to austenite, referred to as Aₛ (A_{f} being the temperature at which the reversion is complete) the deformed object will begin to return to its original configuration.

Many shape memory alloys (SMAs) are known to display stress-induced martensite (SIM). When an SMA sample exhibiting stress-induced martensite is stressed at a temperature above Mₛ (so that the austenitic state is initially stable), but below M_{d} (the maximum temperature at which martensite formation can occur even under stress) it first deforms elastically and then, at a critical stress, begins to transform by the formation of stress-induced martensite. Depending on whether the temperature is above or below Aₛ, the behavior when the deforming stress is released differs. If the temperature is below Aₛ, the stress-induced martensite is stable: but if the temeprature is above Aₛ, the martensite is unstable and transforms back to austenite, with the sample returning (or attempting to return) to its original shape. The effect is seen in almost all alloys which exhibit a thermoelastic martensitic transformation, along with the shape memory effect. However, the extent of the temperature range over which SIM is seen and the stress and strain ranges for the effect vary greatly with the alloy.

In U.S. Patent Application No. 541844, which was filed on 14.10.83 and served to establish a priority date for EP-A-140621, a nickel/titanium/vanadium alloy having SIM over a wide temperature range is disclosed.

Shape memory alloys have found use in recent years in, for example, pipe couplings (such as are described in U.S. Pat. Nos. 4,035,007 and 4,198,081 to Harrison and Jervis), electrical connectors (such as are described in U.S. Pat. No. 3,740,839 to Otte & Fischer), switches (such as are described in U.S. Patent No. 4,205,293), actuators, etc.

Various proposals have also been made to employ shape memory alloys in the medical field. For example, U.S. Pat. No. 3,620,212 to Fannon et al. proposes the use of an SMA intrauterine contraceptive device, U.S. Pat. No. 3,786,806 to Johnson et al. proposes the use of an SMA bone plate, U.S. Pat. No. 3,890,977 to Wilson proposes the use of an SMA element to bend a catheter or cannula, etc.

These medical SMA devices rely on the property of shape memory to achieve their desired effects. That is to say, they relay on the fact that when an SMA element is cooled to its martensitic state and is subsequently deformed, it will retain its new shape; but when it is warmed to its austanitic state, the original shape will be recovered.

However, the use of the shape memory effect in medical applications is attended with two principal disadvantages. First, it is difficult to control the transformation temperatures of shape memory alloys with accuracy as they are usually extremely composition-sensitive, although various techniques have been proposed (including the blending by power metallurgy of already-made alloys of differing transformation temperatures: see U.S. Pat. No. 4,310,354 to Fountain et al.). Secondly, in many shape memory alloys there is a large hysteresis as the alloy is transformed between austenitic and martensitic states, so that reversing of the state of an SMA element may require a temperature excursion of several tens of degrees Celsius. The combination of these factors with the limitation that (a) it is inconvenient to have to engage in any temperature manipulation, and (b) human tissue cannot be heated or cooled beyond certain relatively narrow limits (approximately 0°-60°C for short periods) without suffering temporary or permanent damage is expected to limit the use that can be made of SMA medical devices. It would thus be desirable to develop a way in which the advantageous property of shape memory alloys, i.e. their ability to return to an original shape after relatively substantial deformation, could be used in medical devices without requiring the delicacy of alloying control and/or the temperature control of placement or removal needed by present shape memory alloy devices.

Use of SIM bone-repair plates has been suggested by Oonishi ("TiNi Alloy with Super Elasticity", Orthopaedic Surgery, Vol.32, No.10 (Oct.1981), page 1180); and SIM orthodontic arch wire has been tested by Watanabe (Japanese J. Dental Engineering, Vol.23, No.61 (1982), pages 47-57). Such devices are first attached in their unstressed state to a broken bone or to a tooth within the body, and are subsequently stressed and attached to another part of the bone or another tooth so that the stressed device within the body applies continuous corrective pressure.

The present invention provides a medical device suitable for placement within or proximate to a mammalian body for treatment of the mammalian body, the device comprising
(I) an element formed from a pseudoelastic shape-memory alloy,
   the alloy displaying reversible stress-induced martensite by virtue of being above its As and above its Ms and below its Md at about body temperature (35-40 degrees C), such that it has a stress-induced martensitic state and an austenitic state,
   and the element having (i) a deformed shape when the alloy is in its stress-induced martensitic state and (ii) a different unstressed shape, and
(II) a mechanically-reversible restraint capable of holding the element in its deformed shape to allow it to be positioned within or in proximity to the said body,
wherein the device is constructed so that
(a) the restraint may be reversibly removed from the element while the device is in use within or proximate to the said body at the said body temperature and the element is therefore displaying reversible stress-induced martensite as aforesaid, and
(b) such removal of the restraint causes the element within or proximate to the said body to transform spontaneously from its deformed shape to substantially its unstressed shape without a change in temperature from the said body temperature.

The device according to the present invention, may, for example, be used as a catheter, for example a tracheal catheter, or an intrauterine contraceptive device.

The invention will now be descirbed, by way of example, with reference to the accompanying drawings, wherein:

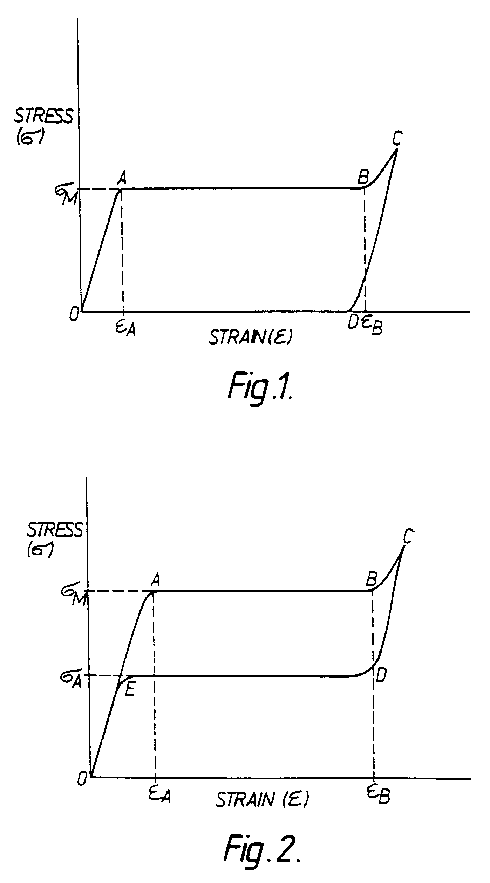

Figures 1 and 2 illustrate the stress-strain behavior of an alloy which exhibits constant stress versus strain behavior due to stress-induced martensite.

The invention will be discussed first by introducing the concept of stress-induced martensite and the effect achievable by its use, and then by examples showing how SIM alloy elements can be substituted for conventional SMA elements in medical devices to achieve the beneficial effect of the invention.

The Figures illustrate the phenomenon of stress-induced martensite by means of stress-strain curves. In both Figure 1 and Figure 2, the alloy is at a temperature between Mₛ and M_{d} so that it is initially austenitic; and it will be assumed for the purposes of this discussion that Mₛ is equal to M_{f}; and Aₛ equal to A_{f}. Figure 1 shows for explanatory purposes the case when the temperature is below Aₛ, so that any martensite formed by the applied stress is stable; while figure 2 shows the case according to the present invention where the temperature is above Aₛ, so that austenite is the only stable phase at zero stress.

In Figure 1, when a stress is applied to the alloy, it deforms elastically along the line OA. At a critical applied stress, σ_{M}, the austenitic alloy begins to transform to (stress-inducted) martensite. This transformation takes place at essentially constant stress until the alloy becomes fully martensitic at point B. From that point on, as further stress is applied, the martensite yields first elastically and then plastically (only elastic deformation is shown at point C). When the stress if released, the martensite recovers elastically to point D, at which there is zero residual stress, but a non-zero residual strain. Because the alloy is below Aₛ, the deformation is not recoverable until heating above Aₛ results in a reversion to austenite. At that point, if the sample is unrestrained, the original shape will be essentially completely recovered: if not it will be recovered to the extent permitted by the restraint. However, if the material is then allowed to recool to the original temperature at which it was deformed (or a temperature where SIM behavior of this type is seen), the stress produced in the sample will be constant regardless of the strain provided that the strain lies within the "plateau" region of the stress-strain curve. That is for a strain between ε_{B} and ε_{A} the stress will be σ_{M} This means that a known, constant force (calculable from σ_{M}) can be applied over a wide (up to 5% or more for certain Ni/Ti alloys) strain range. Thus, though this resembles the conventional shape memory effect, because the alloy shows SIM and is below Aₛ a constant force can be achieved.

In Figure 2, when a stress is applied to the alloy, it deforms elastically along line OA, then by SIM along line AB, and by deformation of the martensite to point C, just as in Figure 1. However, the stress-strain behavior on unloading is significantly different, since the alloy is above Aₛ and the stable phase is therefore austenite. As the stress is removed, the alloy recovers elastically from C to D; then at a critical stress, σ_{A}, the alloy reverts to austenite without requiring a change in temperature. Thus reversion occurs at essentially constant stress. Finally if the stress is removed from the reverted austenite, it recovers elastically along line EO. The recoverable deformation associated with the formation and reversion of stress-induced martensite has been referred to as pseudoelasticity. While σ_{M} may be comparatively high, e.g. 345 MPa (50 ksi), σ_{A} is usually substantially lower, e.g. less than 69 MPa (10 ksi) thereby creating a constant-force spring with an effective working range of about 5% (ε_{B}-ε_{A}). The shape change available in the SMA is thus mechanically, rather than thermally, actuated and controlled, permitting a greater control over a device incorporating it

Suitable alloy for this invention i.e. those displaying stress-induced martensite at temperatures near mammalian body temperature (35°-40°C), may be selected from known SMAs by those of ordinary still in the art, having regard to this disclosure by testing for the existence of the SIM effect at the desired temperature. A particularly preferred alloy is the nickel/titanium/ vanadium alloy referred to previously.

The invention will now be discussed in detail by some Examples of the use of an SIM alloy.

### Example I. Catheters and cannulas

Wilson, in U.S. Patent No. 3,890,977, the disclosure of which is incorporated herein by reference, discloses a catheter or cannula (both being included hereinafter in the word "catheter") made of, or containing, an SMA element to cause all or a portion of the catheter to deplay in a useful form once introduced into a living body.

However, this device has not been commercialized. Possible defects of the device which have prevented commercialization include (i) the inability slowly to emplace the catheter in a desired position when the transition temperature of the alloy is below body temperature (since the SMA element will attempt to revert to its origional shape as it reaches body temperature), thus limiting the ability of the physician to place the device carefully and precisely; or alternatively, if the transition temperature of the alloy is above body temperature, the requirement that the device be heated to a temperature above body temperature to cause recovery and that the device be placed so as not to change shape again when it re-cools (since the body temperature is below the transition temperature); (ii) the inability to remove the device easily; and (iii) the need for controlled temperature storage to prevent premature reversion to austenite of the SMA, with consequent shape change.

The issue of removal of a catheter is especially significant, and not addressed by Wilson. Consider, for example, a tracheal puncture catheter. This should be straight for easy insertion into the trachea through a puncture into the front of the neck, but should curve after insertion so that the flow of air or oxygen through the catheter passes axially down the trachea rather than impinging on the surface of the trachea and damaging it. If a shape memory catheter is used as contemplated by Wilson, it would presumably become austenitic and bend after insertion (see Figures 1a and 1b, and corresponding text, of Wilson). But removal would require either cooling to below the transition temperature (which could easily mean cooling to so low a temperature that the tracheal tissue is damaged), removal in the bent shape (presumably damaging tissue) or forcing the austenitic SMA to straighten to permit direct removal (unlikely to be satisfactory since the austenitic alloys e.g. of Ni/Ti may have yield strengths of 690 MPa (100 ksi) or more, and force sufficient to cause plastic deformation would be required).

If an SIM element is used instead, however, removal can be accomplished almost as easily as insertion. If the catheter is made in a bent shape (as in Wilson), it can be straightened by insertion of a straight pin down the catheter axis, the catheter deforming by the formation of stress-induced martensite. Insertion of the catheter into the trachea is accomplished while the catheter is straight, at whatever rate is desired (permitting easy and accurate placement), and the pin is gradually withdrawn to permit the catheter to take up its desired shape as the martensite reverts to austenite. [It is assumed here that the stress-strain curve of the alloy at the temperature of use is of the form of Figure 2, so spontaneous reversion occurs on removal of the stress induced by the pin]. When removal is desired, it may be achieved simply by the gradual insertion of the pin, straightening the catheter and permitting easy withdrawal. Insertion of the catheter into the body and pin removal may, of course, take place simultaneously if desired. as may pin reinsertion and removal of the catheter from the body.

### Example II. IUDS

Fannon et al, in U.S. patent No. 3,620,212, the disclosure of which is incorporated herein by reference, discloses an intrauterine contraceptive device (an IUD) proposed to be formed of a shape memory alloy. The device is suggested to be deformed in the martensitic phase (the transition temperature being below the temperature of the uterus), and the deformed device insulated with, e.g., wax and inserted it. Removal is contemplated only by using two SMA elements in opposition, the higher temperature one being martensitic at body temperature but strong enough so that, if heated, it will overcome the lower temperature element and deform the IUD back to a removable shape. The heating contemplated is electrical. The storage problem discussed in Example II also exists here, so that the device must be stored below its transition temperature.

By the use of an SIM element. however, these disadvantages may be overcome. Again, assume that the alloy is SIM pseudoelastic, i.e. that it has the stress-strain curve of Figure 2. Then an IUD may be formed into the desired shape in the austenitic state, and deformed by compression into a tubular placement device (the deformation being such that the strain levels lie within the "plateau" of the stress-strain curve). When the placement device is inserted into the uterus, the IUD may be deployed by extrusion of the IUD from the placement device. Deployment is then controlled but immediate, so that the physician may satisfy himself with placement. Removal is the reversal of placement. The placement device is inserted into the uterus, the IUD deformed by withdrawal into the placement device, and the placement device withdrawn. Temperature control is not required.

### Example III. Coil stents and filters

The use of tubular coiled wire stent grafts has been discussed in the medical literature since 1969. Although the coils helped maintain patency of the vessels in which they were placed, they were difficult of insertion unless narrow enough to significantly narrow the lumen of the vessel. Recently it has been proposed, see Radiology, v. 147, pp. 259-60 and pp. 261-3 (1983), the disclosures of which are incorporated herein by reference, to use SMA wire to form these tubular coils. The wire, which has a transformation temperature below body temperature, is introduced through a catheter after being straightened in its martensitic state. When the wire is heated. the coil re-forms.

Because of the difficulty of controlling the transformation temperature accurately, it has proved necessary to cool the straightened wire during insertion and/or to heat the wire to form the coil after insertion. These procedures add to the complexity of the operation.

If an SIM pseudoelastic wire is used to form the coil, which is then isothermally deformed by loading into a catheter, then the need for temperature control is avoided. The wire remains straight when in the catheter, but re-forms the coil spontaneously when it is extruded from the catheter. Accurate placement is thus readily obtainable, since there is no urgency as might be required with a conventional shape memory effect element.

It has similarly been proposed to use SMA wire to form a filter for emplacement by catheter in the vena cava to trap blood clots. The filter is formed in the austenitic state, the wire straightened in the martensitic state and inserted, and the filter re-forms on warming. Just as for the coil stents discussed above, the use of an SIM pseudoelastic wire would greatly simplify manufacture and insertion of such a vena cava filter, permitting accurate placement with no need for urgency or temperature manipulation.

From the foregoing, it is clear that, in a situation where narrow temperature differences are available or preferable, as often is the case in medical applications, mechanically constrained shape change is a much more useful solution than heat actuated shape change. It offers a degree of control heat actuation does not, it offers easier alloy composition control, it eases mating part tolerance requirements, and it offers simple mechanical reversal at minimal stress levels, all without heating, cooling or insulation complications.

## Claims

1. A medical device suitable for placement within or proximate to a mammalian body for treatment of the mammalian body, the device comprising
(I) an element formed from a pseudoelastic shape-memory alloy,
the alloy displaying reversible stress-induced martensite by virtue of being above its As and above its Ms and below its Md at about body temperature (35-40 degrees C), such that it has a stress-induced martensitic state and an austenitic state,
and the element having (i) a deformed shape when the alloy is in its stress-induced martensitic state and (ii) a different unstressed shape, and
(II) a mechanically-reversible restraint capable of holding the element in its deformed shape to allow it to be positioned within or in proximity to the said body,
wherein the device is constructed so that
(a) the restraint may be reversibly removed from the element while the device is in use within or proximate to the said body at the said body temperature and the element is therefore displaying reversible stress-induced martensite as aforesaid, and
(b) such removal of the restraint causes the element within or proximate to the said body to transform spontaneously from its deformed shape to substantially its unstressed shape without a change in temperature from the said body temperature.

2. A device as claimed in claim 1, in which the restraint is hollow, and the shape memory alloy element is deformed in such a way that it is compressed transversely, and is positioned within the restraint, the restraint preventing transverse expansion of the element.

3. A device as claimed in claim 2, in which the restraint is a catheter.

4. A device as claimed in claim 2, in which the shape memory alloy element is an intrauterine contraceptive device.

5. A device as claimed in claim 3, in which the shape memory alloy element is a filter for a blood vessel.

6. A device as claimed in claim 1, in which the shape memory alloy element is tubular, and the restraint is positioned within the shape memory alloy element to deform it.

7. A device as claimed in claim 6, in which the shape memory alloy element is a catheter.

## Patentansprüche

1. Medizinische Einrichtung, geeignet zum Anordnen im Inneren oder in der Nähe eines Säugetierkörpers für die Behandlung des Säugetierkörpers, wobei die Einrichtung folgendes aufweist:
I) ein Element, das aus einer pseudoelastischen Formerinnerungslegierung gebildet ist, wobei die Legierung sich als reversibler spannungsinduzierter Martensit erweist aufgrund des Umstandes, daß sie über ihrer As-Temperatur und über ihrer Ms-Temperatur und unter ihrer Md-Temperatur ungefähr auf Körpertemperatur (35 bis 40 °C) ist, so daß sie einen spannungsinduzierten martensitischen Zustand und einen austenitischen Zustand hat,
und wobei das Element (i) eine deformierte Gestalt hat, wenn die Legierung in ihrem spannungsinduzierten martensitischen Zustand ist, und (ii) eine andere entspannte Gestalt hat, und
II) eine mechanisch reversible Halteeinrichtung, die in der Lage ist, das Element in seiner deformierten Gestalt zu halten, um es zu ermöglichen, daß es im Inneren oder in der Nähe des Körpers positioniert wird,
wobei die Einrichtung so aufgebaut ist, daß
a) die Halteeinrichtung von dem Element reversibel entfernt werden kann, während die Einrichtung im Inneren oder in der Nähe des Körpers bei der Körpertemperatur verwendet wird, und das Element sich deshalb als reversibler spannungsinduzierter Martensit erweist, wie es oben angegeben ist, und
b) ein derartiges Entfernen der Halteeinrichtung dazu führt, daß das Element im Inneren oder in der Nähe des Körpers spontan aus seiner deformierten Gestalt in seine im wesentlichen entspannte Gestalt ohne Temperaturänderung von der Körpertemperatur übergeht.

2. Einrichtung nach Anspruch 1,
bei der die Halteeinrichtung hohl ist und das Element aus einer Legierung mit Formerinnerungsvermögen in einer solchen Weise deformiert ist, daß es in Querrichtung komprimiert ist, und innerhalb der Halteeinrichtung positioniert ist, wobei die Halteeinrichtung eine Querausdehnung des Elementes verhindert.

3. Einrichtung nach Anspruch 2,
bei der die Halteeinrichtung ein Katheter ist.

4. Einrichtung nach Anspruch 2,
bei der das Element aus einer Legierung mit Formerinnerungsvermögen eine Intrauterinspange ist.

5. Einrichtung nach Anspruch 3,
bei der das Element aus einer Legierung mit Formerinnerungsvermögen ein Filter für ein Blutgefäß ist.

6. Einrichtung nach Anspruch 1,
bei der das Element aus einer Legierung mit Formerinnerungsvermögen rohrförmig ist und die Halteeinrichtung innerhalb des Elementes aus einer Legierung mit Formerinnerungsvermögen positioniert ist, um es zu deformieren.

7. Einrichtung nach Anspruch 6,
bei der das Element aus einer Legierung mit Formerinnerungsvermögen ein Katheter ist.

## Revendications

1. Dispositif médical convenant pour être mis en place à l'intérieur ou à proximité de l'organisme d'un mammifère en vue du traitement de l'organisme du mammifère, ce dispositif comprenant :
(I) un élément formé en un alliage pseudo-élastique à mémoire de forme, cet alliage présentant de la martensite réversible induite par des contraintes du fait qu'il se trouve au-dessus de sa température Aₛ et au-dessus de sa température Mₛ et au-dessous de sa température M_{d} approximativement à la température du corps (35-40°C), si bien qu'il a un état martensitique induit par des contraintes et un état austénitique,
l'élément ayant (i) une configuration déformée lorsque l'alliage se trouve dans son état martensitique induit par des contraintes et (ii) une forme différente en l'absence de contraintes, et
(II) une entrave mécaniquement réversible capable de maintenir l'élément dans sa configuration déformée pour permettre son positionnement à l'intérieur ou à proximité du corps,
le dispositif étant construit de manière que
(a) l'entrave puisse être retirée de façon réversible de l'élément tandis que le dispositif est en position d'utilisation à l'intérieur ou à proximité du corps à la température de ce corps et l'élément présente donc une martensite réversible induite par des contraintes comme indiqué ci-dessus, et
(b) ce retrait de l'entrave permettant à l'élément dans le corps ou à proximité du corps de se transformer spontanément en passant de sa configuration déformée à sa forme sensiblement sans contrainte, sans changement de température à partir de la température du corps en question.

2. Dispositif suivant la revendication 1, dans lequel l'entrave est creuse et l'élément en alliage a mémoire de forme est déformé de façon telle qu'il soit comprimé transversalement, et est mis en place dans l'entrave, cette dernière empêchant une expansion transversale de l'élément.

3. Dispositif suivant la revendication 2, dans lequel l'entrave est un cathéter,

4. Dispositif suivant la revendication 2, dans lequel l'élément en alliage à mémoire de forme est un dispositif Contraceptif intra-utérin.

5. Dispositif suivant la revendication 3, dans lequel l'élément en alliage à mémoire de forme est un filtre pour un vaisseau sanguin.

6. Dispositif suivant la revendication 1, dans lequel l'élément en alliage à mémoire de forme est tubulaire et l'entrave est disposée dans l'élément en alliage à mémoire de forme afin de déformer cet élément.

7. Dispositif suivant la revendication 6, dans lequel l'élément en alliage à mémoire de forme est un cathéter.
